# EUROPEAN PATENT SPECIFICATION

(11) **EP 3 816 606 B1**
(45) Date of publication and mention of the grant of the patent: **22.02.2023**
(21) Application number: 20203625.7
(22) Date of filing: 23.10.2020
(51) Int. Cl.: G01N 3/24, G01N 19/02, G01N 33/18

(54) **APPARATUS AND METHOD FOR THE ANALYSIS OF SNOWPACK SAMPLES**
VORRICHTUNG UND VERFAHREN FÜR DIE ANALYSE VON SCHNEEDECKENPROBEN
DISPOSITIF ET PROCÉDÉ D'ANALYSE D'ÉCHANTILLONS DE MANTEAU NEIGEUX

(30) Priority: 30.10.2019 IT 201900020086
(43) Date of publication of application: 05.05.2021
(73) Proprietor: Bridgestone Europe NV/SA, 1932 Zaventem (BE)
(72) Inventor: Rodriquez, Giuseppe, 00128 Roma (IT); Agoretti, Pasquale, 00128 Roma (IT); Maeda, Shimpei, 00128 Roma (IT)
(74) Representative: Marchetti, Alessio

(56) References cited:
- JP-A- 2004 125 620
- JP-A- 2004 257 892
- SU-A1- 885 865
- DAVID M MC CLUNG: "DIRECT SIMPLE SHEAR TESTS ON SNOW AND THEIR RELATION TO SLAB AVALANCHE FORMATION", JOURNAL OF GLACIOLOGY, vol. 19, no. 81, 1 January 1977 (1977-01-01), pages 101-109, XP055715921,

## Description

### TECHNICAL SECTOR

The present invention relates to a portable apparatus for subjecting snowpack samples to analysis and to a related method for subjecting snowpack samples to analysis.

### STATE OF THE ART

In order to meet the demands of users to have pneumatic tyres that are suitable for all seasons, the requirement is increasingly being felt to improve the performance offered by pneumatic tyres on snow.

The main factors that affect the ability of a vehicle to develop forces of traction on a snowpack are, in fact, the characteristics of the pneumatic tyres which are fitted to the vehicle and the properties of the snowpack.

With regard to the ability of a pneumatic tyre to generate forces on a snow-covered surface, this depends upon the rubber compound and the structure of the pneumatic tyre, in particular a pneumatic tyre tread (by the dimensions, design and geometrical characteristics of the blocks and of the tread grooves and by the ability thereof to trap layers of snow).

As regards, by contrast, the contribution due to the characteristics of the snowpack, it was verified that this contribution depends primarily upon the shear strength of the volumes of snow trapped within the grooves of the tread. As is known, the snowpack is formed from the superposition of multiple layers (fresh snow, weak/brittle snow, compact/resistant snow, ice) deriving from subsequent additions of snow; each layer is characterized by a content of water (or humidity), by the density thereof and by the presence of snow having specific structures and crystal dimensions. The contribution to the forces exerted by the pneumatic tyre depends heavily upon the density of the volumes of snow trapped within the grooves of the tread and, consequently, is linked to the mechanical and morphological characteristics of the layer(s) of snow involved in said volumes.

The set of characteristics of the different layers of snow and the environmental parameters (temperature, humidity, residence time) determines the mechanical properties of each layer; clearly the mechanical properties of each layer are not predictable by means of indirect measurements.

It is therefore clear that, during the research and development phases when assessing the performance offered by pneumatic tyres when transiting over a snowpack, it is extremely important to be able to compare the performance obtained on snowpacks with similar characteristics. There is therefore an increasing need to evaluate the mechanical characteristics of snowpacks in order to be able to compare the performance offered by the different pneumatic tyres.

JP2004257892A discloses an apparatus with sample compression means and sample shearing means. The sample compression means being provided with container compression movement means with upper and lower container sections that can compress snow accommodated in the inside and with a compression load measuring instrument for measuring compression load. The sample shearing means are provided with container traverse means for relatively, horizontally moving the upper and lower container sections shear the compressed snow; and a shearing load measuring apparatus for measuring shearing load.

### DESCRIPTION OF THE INVENTION

The aim of the present invention is to provide a portable apparatus for subjecting snowpack samples to analysis that is free from the disadvantages of the state of the art and that is, in particular, easy and inexpensive to implement.

A further aim of the present invention is to provide a method for subjecting snowpack samples to analysis that is free from the disadvantages of the state of the art and, in particular, that is easy and inexpensive to implement.

According to this invention, a portable apparatus is provided for the analysis of snowpack samples and a related method for subjecting snowpack samples to analysis, as set out in the appended claims.

### BRIEF DESCRIPTION OF THE DRAWINGS

The present invention is now described with reference to the attached drawings, which illustrate several non-limiting exemplary embodiments, wherein:
- Figure 1 is a prospective and exploded view of a portable apparatus for subjecting snowpack samples to analysis that is implemented in accordance with this invention;
- Figure 2 is a partial and section view of the portable apparatus in Figure 1; and
- Figure 3 is a prospective view of a detail of the portable apparatus of Figure 1.

### PREFERRED EMBODIMENTS OF THE INVENTION

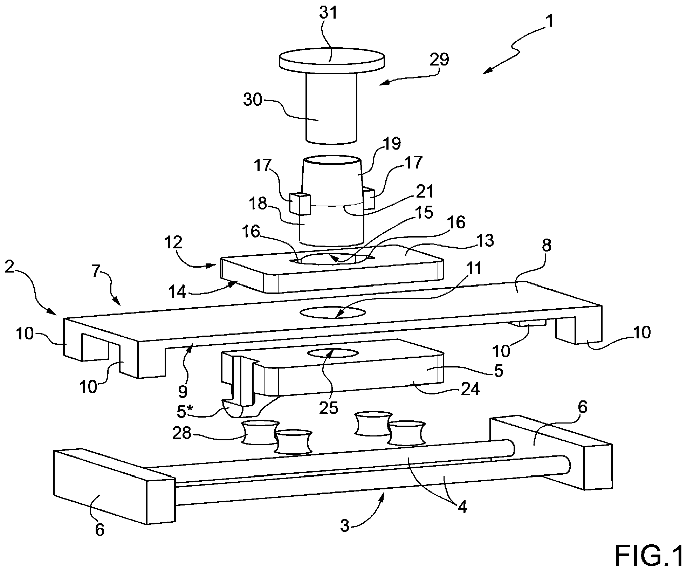

In Figure 1, the number 1 indicates in the entirety thereof, a portable apparatus for subjecting snowpack samples C to analysis.

In particular, the device 1 comprises a structure 2 which in turn comprises a fixed guide structure 3 defined by two linear guides 4 that are parallel therebetween and two head elements 6. As better described in the following discussion, a carriage 5 slides on linear guides 4.

The two linear guides 4 are connected, at the ends thereof, to the two head elements 6 intended to act as a limit switch for the carriage 5.

The structure 2 comprises a plate 7 with a parallelepiped shape having a reduced thickness (on the order of 5-10 mm) and presenting a flat upper surface 8 and a flat lower surface 9. At the vertices of the lower flat surface 9, four appendages 10 are provided for the connection by appropriate fastening means (known and not described in detail) with the two head elements 6.

The plate 7 is equipped with a through opening 11 formed at a substantially central position and provided with an X-axis. The aperture 11 is defined by a cylindrical surface with a diameter of between 20 and 50 mm, preferably between 25 and 35 mm.

According to a preferred variant, the diameter of the aperture 11 is 30 mm.

The structure 2 also includes an adapter 12 defined by a parallelepiped shaped plate with a thickness of between 5 and 15 mm. According to a preferred variant, the thickness of the adapter 12 is 10 mm. The adapter 12 has a flat upper surface 13 and a flat lower surface 14. The lower surface 14 is arranged in contact with (lies upon) the upper surface 8.

The adapter 12 is connected, by appropriate fastening means (known and not described in detail) to the plate 7. The adapter 12 preferably has, in plan view, dimensions that approximate, by default, the dimensions of the plate 7. The adapter 12 is also provided with a through opening 15 formed at a substantially central and coaxial position with respect to the X-axis (and therefore coaxial, in use, to the opening 11). The aperture 15 is defined by a cylindrical surface with the same diameter as the opening 11, i.e., a diameter of between 20 and 50 mm, preferably of between 25 and 35 mm. According to a preferred variant, the diameter of the aperture 15 is 30 mm.

Furthermore, the adapter 12 is provided with two grooves 16 intended to receive a respective lateral rib 17 of a sampling element 18, as better described in the following discussion. The two grooves 16 are formed at the opening 15. In particular, the grooves 16 are formed at diametrically opposite ends (with respect to the X-axis) of the opening 15. The grooves 16 are formed at the upper surface 16. Each groove 16 is defined by a surface with a substantially flat base which is joined to a back wall (curved or flat) and two side walls, which are also substantially flat.

The sampling element 18 is intended both to produce the snowpack core sample C to be analyzed and to receive the snowpack sample C during the analysis step. According to a preferred variant, the sampling element 18 is made of a plastic or metallic material; the sampling element 18 is preferably obtained by means of 3D molding techniques.

The sampling element 18 is defined by a tubular body 19 having an inner surface 20 with cylindrical symmetry around an X'-axis. The inner surface 20 has a diameter of between 15 and 45 mm; preferably the diameter of the inner surface 20 is between 20 and 30 mm.

The tubular body 19 is divided into a coring portion 19* and an analysis portion 19** joined together at an outer edge 21. The coring portion 19* and the measurement portion 19** have respective substantially uniform extensions along the X'-axis. According to alternative embodiments, the portions 19* and 19** may be uniform or non-uniform, symmetrical or non-symmetrical, therebetween.

Preferably (but not exclusively), the coring portion 19* has an extension along the X'-axis of between 15 and 30 mm, preferably equal to 22 mm. In this case, the dimensions of the coring portion 19* make it possible to isolate samples C of a single layer from the layers that define the snowpack. In this way it is possible, by means of differing samplings, to take samples C of the individual layers of snowpack that are responsible for the performance of the pneumatic tyres on snow and evaluate the shear resistance thereof.

The analysis portion 19** has an outer surface 22 that is cylindrical and coaxial with respect to the X'-axis.

The coring portion 19* has instead an outer truncated-cone surface 23 that is coaxial to the X'-axis.

In other words, the outer surface 23 gradually widens towards the analysis portion 19**. The cylindrical outer surface 22 therefore has a diameter equal to the larger diameter of the outer truncated-cone surface 23. The particular shape of the coring portion 19* makes it possible to penetrate the snowpack, isolate, detach and retain the snowpack sample C within the area defined by the inner surface 20.

Furthermore, the sampling element 18 carries, and is connected to, the two lateral ribs 17 which extend laterally from the outer surfaces 22, 23. The lateral ribs 17 are substantially arranged at the edging 21. The lateral ribs 17 are arranged at the extremity therebetween, diametrically opposite (with respect to the X'-axis) the outer surfaces 22, 23. The lateral ribs 17 have a shape that is complementary to the shape of the grooves 16. In particular, each lateral rib 17 is provided with respective opposing bottom surfaces which are arranged, when in use, in abutment on the bottom surface of the respective groove 16, in such a way that the distance between the lower end of the sampling element 18 and the upper end of the carriage 5 is between 0.1 and 3 mm. It is important to highlight that both ends of the lateral ribs 17 have a shape that is complementary to the shape of the grooves 16. In other words, both the ends of the lateral ribs 17 facing the coring portion 19* and the ends of the lateral ribs 17 facing the analysis portion 19** have complementary shapes to the shape of the grooves 16 and are intended to engage, in use, with the grooves 16.

The carriage 5 is defined by a box-like body 24 provided with a seat 25 for partially receiving the snowpack sample C of the snowpack and is arranged, in use, below the plate 7. In use, the carriage 5 is housed in such a way that the seat 25 is turned towards and directly facing the plate 7. The seat 25 is defined by a circular bottom surface 26 and by a cylindrical lateral surface 27 having a diameter that approximates the diameter of the inner surface 20. The seat 25 has a variable height of between 1 mm and 20 mm; preferably, the height of the seat 25 is between 8 and 15 mm. The height of the seat 25 is modified (adjusted) by means of the insertion of a number of disc shims (not shown) of suitable height within the seat 25 itself. Underneath, the carriage 5 is connected to four bearings 28 which allow for the sliding of the carriage 5 itself on two rails 3.

A front end of the carriage 5 is provided with an appendix 5* that protrudes such as to be interposed, when in use, between the two rails 3. In a front surface of the box-like body 24 there is a groove which together with the appendix 5* defines a housing that is intended to house a load cell for detecting the shear strength of the snowpack sample C.

According to further variants, as an alternative to the load cell, the apparatus 1 may comprise a piezoelectric force sensor or a magnetic force sensor or a hydraulic force sensor.

The apparatus 1 comprises a compressive member, in particular a piston 29, having a cylindrical body 30 that is intended to slide within the sampling element 18 and an upper handle 31 (or knob).

Clearly, the cylindrical body 30 is provided with a cylindrical outer surface wherein the diameter thereof approximates, by default, the diameter of the inner surface 20.

Finally, the apparatus comprises an actuating device 32, in particular an electric motor, which is connected to the carriage 5 and that is suitable for sliding the carriage 5 on the rails 3. According to a first variant, the actuating device 32 is arranged externally to the box-like body 24, within a dedicated housing. According to a further variant, the actuating device 32 is housed within the box-like body 24.

Advantageously, a further load cell is provided in order to detect the compression force exerted by the piston 29 upon the snowpack sample C; the further load cell is preferably housed on the piston 29.

The following describes the method for using the portable apparatus 1 in subjecting to analysis snowpack samples C, as described heretofore.

In the first place, the operator proceeds manually to implementing the sampling (coring) of the snowpack sample C for analysis. The snowpack sample C is taken in penetrating into the snowpack using the coring portion 19*, such that the snowpack sample C engages with the seat defined, within the coring portion 19* itself, by the inner surface 20. At this stage, the analysis portion 19** is used by the operator to grasp the sampling element 18, whilst the coring portion 19* serves as a core barrel; the outer truncated-cone surface 23 in fact allows for the penetration into the snowpack whilst the inner surface 20 protects and houses the snowpack sample C.

When the sampling element 18 reaches a depth such as to encompass the entire thickness of the layer of snow to be analyzed, the sampling step is concluded.

Once the sampling step has been completed, the sampling element 18 is extracted from the snowpack and inserted into the structure 2, in such a way as to coincide with the X' and X axes. The sampling element 18 acts, in this case, as a centering pin.

According to alternative therebetween embodiments and as a function of the snowpack layer to be analyzed, the analysis portion 19** or else the coring portion 19* is inserted within the openings 11 and 15.

According to a first variant, the analysis portion 19** is inserted within the openings 11 and 15; in particular, the analysis portion 19** slides inside the openings 11 and 15 until it stops when each lateral rib 17 has engaged with the respective groove 16. The analysis portion 19** protrudes (juts out from the plate 7 downwards) such as to partially fill the free space between the plate 7 and the carriage 5. It is important to highlight that the analysis portion 19** (in particular, a bottom surface of the analysis portion 19**) is arranged in the vicinity of a flat upper surface of the carriage 5, but not in contact (i.e., not resting in contact) with the carriage 5. In other words, during the analysis step, the analysis portion 19** and the carriage 5 are separated by a gap (slit or slot) of a few mm, in particular the dimensions of said interstice are between 0.1 and 3 mm.

The carriage 5 is located below the sampling element 18, such that the seat 25 is coaxial to the openings 11 and 15. In other words, the seat 25 is facing the sampling element 18 such as to partially receive the snowpack sample C.

Subsequently, the piston 29 is inserted and made to slide, inside the sampling element 18, by an operator. Preferably, the pressure exerted by the operator on the snowpack sample C by means of the piston 29 is detected by means of the further load cell housed on the piston 29.

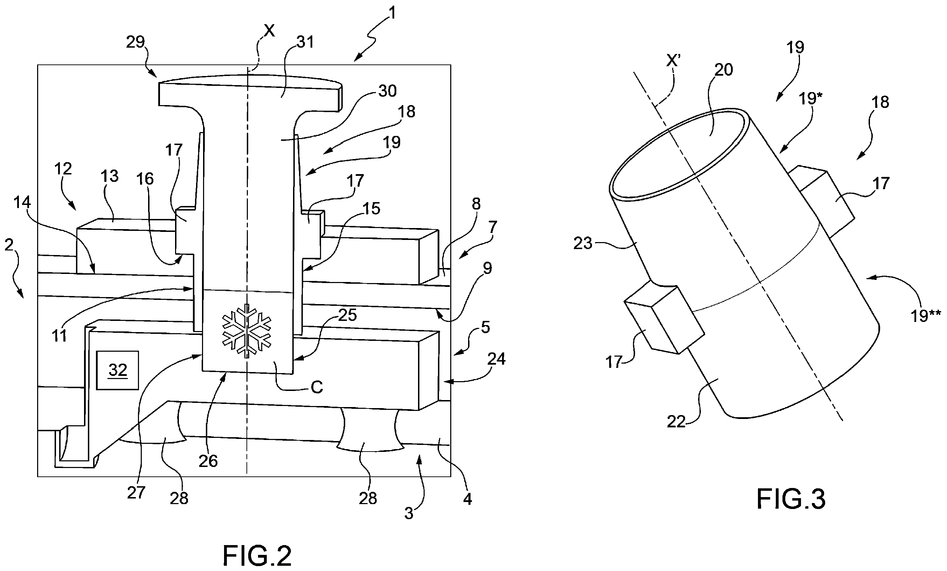

The snowpack sample C slides downwards (i.e., towards the carriage 5) until the load, detected by the load cell at the piston head 29, reaches a target compression value, determined at a preliminary stage of the analysis. In the position wherein the load, detected by the load cell in the piston head 29, has reached the target compression value shown in Figure 2, the snowpack sample C is partially housed within the seat 25 and partially housed within the analysis portion 19**.

Finally, the actuating device 32 is driven in order to allow the sliding of the carriage 5 upon the rails 3; the shear strength of the snowpack sample C is detected by means of the load cell.

According to a further variant, the coring portion 19*, after the actual coring step, is inserted directly within the openings 11 and 15; in particular, the coring portion 19* slides inside the openings 11 and 15 until it stops when each lateral rib 17 has engaged with the respective groove 16. The coring portion 19* protrudes (juts out from the plate 7 downwards) such as to partially fill the free space between the plate 7 and the carriage 5. It is important to highlight that the coring portion 19* (in particular, a bottom surface of the coring portion 19*) is arranged in the vicinity of a flat upper surface of the carriage 5, but not in contact (i.e., not resting in contact) with the carriage 5.

In other words, during the analysis step, the coring portion 19* and the carriage 5 are separated by an interstice (slit or slot) of a few mm, in particular the dimensions of said interstice are between 0.1 and 3 mm.

The carriage 5 is located below the sampling element 18, such that the seat 25 is coaxial to the openings 11 and 15. In other words, the seat 25 is facing the sampling element 18 such as to partially receive the snowpack sample C.

Subsequently, the piston 29 is inserted and made to slide, inside the sampling element 18, by an operator. Preferably, the pressure exerted by the operator on the snowpack sample C by means of the piston 29 is detected by means of the further load cell housed on the piston 29.

The snowpack sample C slides downwards (i.e., towards the carriage 5) until the load, detected by the load cell at the piston head 29, reaches a target compression value, determined at a preliminary stage of the analysis. In the position wherein the load, detected by the load cell in the piston head 29, has reached the target compression value shown in Figure 2, the snowpack sample C is partially housed within the seat 25 and partially housed within the coring portion 19*.

Finally, the actuating device 32 is driven in order to allow the sliding of the carriage 5 upon the rails 3; the shear strength of the snowpack sample C is detected by means of the load cell.

It has been shown experimentally that the measurement of the shear strength of the snowpack sample C, performed by means of the portable apparatus 1 described in the foregoing discussion, is extremely reliable; the measurement by means of the load cell provides comparable values to shear strength values measured using other techniques.

The portable apparatus 1 is used to measure the shear strength of snowpack samples C, with the same characteristics, taken from subsequent corings and in exerting different pressure values on the sample C. Alternatively, it is possible to exert a constant pressure and to measure the shear strength of snowpack samples C taken from subsequent corings from different layers of the snowpack.

It is important to note that the method for subjecting the snowpack samples C to analysis, as described heretofore, do not provide for any transfer of the snowpack sample C between the coring/withdrawal and the actual analysis step. In this way, in reducing the manipulation of the snowpack sample C by operators, it is possible to not affect the properties of the snowpack sample C.

The apparatus 1, thus far described, has numerous advantages. Firstly, the apparatus 1 described above makes it possible to significantly reduce manipulation of the snowpack samples C, from the coring step to the actual analysis step. In fact, the apparatus 1 as described heretofore makes it possible, after the coring step, for the sampling element 18 to be overturned, but not further transferred or handled.

In addition, the apparatus 1 has extremely compact dimensions that make it possible to move it in an easy manner and to perform *in-situ* analysis, as well as allowing for a significant improvement in the repeatability of the analysis allowing for the withdrawal of perfectly uniform snowpack samples C; finally, the apparatus 1 is used to evaluate, one at a time, individual layers of the snowpack in an extremely reliable way.

### LIST OF REFERENCE NUMBERS IN THE FIGURES

- 1: portable apparatus
- 2: structure
- 3: guide system
- 4: linear guide
- 5: carriage
- 5*: extension
- 6: head elements
- 7: plate
- 8: upper surface
- 9: lower surface
- 10: extensions
- 11: opening
- 12: adapter
- 13: upper surface
- 14: lower surface
- 15: opening
- 16: grooves
- 17: lateral ribs
- 18: sampling element
- 19: tubular body
- 20: inner surface
- 21: outer edging
- 22: outer surface
- 23: outer surface
- 24: body
- 25: seat
- 26: bottom surface
- 27: lateral surface
- 28: bearings
- 29: piston
- 30: cylindrical body
- 31: handle
- 32: electric motor
- X': axis
- X: axis
- C: snowpack
- 19*: coring portion
- 19**: analysis portion

## Claims

1. Portable apparatus (1) for subjecting a snowpack sample (C) to analysis comprising
a structure (2) provided in turn with:
- a fixed guide structure (3) defined by two linear guides (4);
- a plate (7) provided with a first through opening (11) having cylindrical symmetry about a first axis (X);
- a carriage (5) that slides on the linear guides (4) and that is provided with a seat (25) that is intended to house, at least partially, the snowpack sample (C) and facing, in use, the first through opening (11);
a carriage (5) actuating device (32); and
an extractable sampling element (18), implemented by means of a tubular body (19) having an inner surface (20) with cylindrical symmetry about a second axis (X') which defines a seat for receiving the snowpack sample (C);
the apparatus is **characterized in that** the extractable sampling element (18) is divided into a coring portion (19*) and an analysis portion (19**); wherein the coring portion (19*) has an outer truncated-cone surface (23) that is suitable for penetrating into the snowpack in order to isolate and retain the sample (C) within the seat defined by the inner surface (20); and wherein the analysis portion (19**) has an outer cylindrical surface (22) having a diameter that substantially approximates the diameter of the first through opening (11).

2. Apparatus according to claim 1, wherein the sampling element (18) comprises a number of lateral ribs (17) which protrude from the lateral surface (22, 23); and wherein the structure (2) comprises a number of grooves (16), each one of which is intended to receive a respective lateral rib (17) during the snowpack sample (C) analysis step.

3. Apparatus according to claim 1 or 2, wherein the coring portion (19*) and the analysis portion (19**) are joined together at an outer edge (21); and wherein the sampling element (18) comprises a number of lateral ribs (17) which protrude from the lateral surface (22, 23) at the outer edge (21).

4. Apparatus according to any one of the preceding claims, wherein the inner surface (20) has a diameter of between 15 and 45 mm; preferably the diameter of the inner surface (20) is between 20 and 30 mm.

5. Apparatus according to any one of the preceding claims, wherein the outer cylindrical surface (22) has a diameter which coincides with the larger diameter of the outer truncated-cone surface (23).

6. Apparatus according to any one of the preceding claims, wherein the coring portion (19*) has an extension along the second axis (X') of between 15 and 30 mm, such as to isolate samples (C) of a single layer from between those layers that define the snowpack.

7. Apparatus according to any one of the preceding claims and comprising a member (29) for the compression of the snowpack sample (C) having a cylindrical body (30) that is intended to slide inside the sampling element (18).

8. Apparatus according to claim 7, wherein the compression member (29) comprises a first load cell for measuring the compressive force exerted on the snowpack sample (C).

9. Apparatus according to any one of the preceding claims, wherein the carriage (5) comprises a second load cell for measuring the shear strength of the snowpack sample (C).

10. Apparatus according to any one of the preceding claims, wherein the structure (2) comprises an adapter (12) that is connected to the plate (7) and provided with a second through opening (15) that is coaxial to the first axis (X).

11. Apparatus according to claim 10 when it depends on claim 2, wherein the grooves (16) are formed in the adapter (12).

12. Apparatus according to any one of the preceding claims, wherein during the snowpack sample (C) analysis step, the end of the analysis portion (19**) and the carriage (5) are separated by an interstice with dimensions of between 0.1 and 3 mm.

13. Method for subjecting a snowpack sample (C) to analysis by means of a portable apparatus (1) implemented according to any one of the claims from 1 to 12 and comprising:
a step for the withdrawal, by means of the coring portion (19*), of a snowpack sample (C);
a step for the insertion of the sampling element (18) within the through opening (11);
a step of applying a pressure to the snowpack sample (C) in order to move it from a withdrawal position, wherein the sample (C) is entirely housed inside the sampling element (18), to an analysis position, wherein the sample (C) is partially housed within the sampling element (18) and partially within the seat (25);
a step for controlling, by means of the actuation device (32), the movement of the carriage (5); and
a step for measuring the shear strength of the snowpack sample (C).

14. Method according to claim 13, wherein the insertion step provides for the insertion of the coring portion (19*) within the through opening (11).

15. Method according to claim 14, wherein the insertion step provides for the insertion of the analysis portion (19**) within the through opening (11).

16. Method according to any one of the claims from 13 to 15, wherein no further steps are provided for the processing or transferring of the snowpack sample (C) between the withdrawal step and the insertion step.

## Patentansprüche

1. Tragbare Vorrichtung (1), um eine Schneedeckenprobe (C) einer Analyse zu unterziehen, umfassend
eine Struktur (2), die wiederum mit Folgendem versehen ist:
- einer festen Führungsstruktur (3), die durch zwei Linearführungen (4) definiert ist;
- einer Platte (7), die mit einer ersten Durchgangsöffnung (11) versehen ist, die eine zylindrische Symmetrie um eine erste Achse (X) aufweist;
- einem Schlitten (5), der auf den Linearführungen (4) gleitet und der mit einem Sitz (25) versehen ist, der dazu bestimmt ist, die Schneedeckenprobe (C) mindestens teilweise zu beherbergen und während der Verwendung der ersten Durchgangsöffnung (11) zugewandt zu sein;
eine Schlitten (5) Betätigungsvorrichtung (32); und
ein ausziehbares Probenahmeelement (18), das mittels eines rohrförmigen Körpers (19) verwirklicht ist, der eine Innenfläche (20) mit einer zylindrischen Symmetrie um eine zweite Achse (X') aufweist, die einen Sitz zum Aufnehmen der Schneedeckenprobe (C) definiert;
wobei die Vorrichtung **dadurch gekennzeichnet ist, dass** das ausziehbare Probenahmeelement (18) in einen Kernbohrungsabschnitt (19*) und einen Analyseabschnitt (19**) unterteilt ist; wobei der Kernbohrungsabschnitt (19*) eine kegelstumpfförmige Außenfläche (23) aufweist, die geeignet ist, in die Schneedecke einzudringen, um die Probe (C) innerhalb des durch die Innenfläche (20) definierten Sitzes zu isolieren und zu halten; und wobei der Analyseabschnitt (19**) eine zylindrische Außenfläche (22) aufweist, die einen Durchmesser aufweist, der sich im Wesentlichen dem Durchmesser der ersten Durchgangsöffnung (11) nähert.

2. Vorrichtung nach Anspruch 1, wobei das Probenahmeelement (18) eine Anzahl von seitlichen Rippen (17) umfasst, die von der Seitenfläche (22, 23) hervorvorstehen; und wobei die Struktur (2) eine Anzahl von Nuten (16) umfasst, von denen jede dazu bestimmt ist, eine jeweilige seitliche Rippe (17) während des Schneedeckenproben-(C) Analyseschritts aufzunehmen.

3. Vorrichtung nach Anspruch 1 oder 2, wobei der Kernbohrungsabschnitt (19*) und der Analyseabschnitt (19**) an einer Außenkante (21) miteinander verbunden sind; und wobei das Probenahmeelement (18) eine Anzahl von seitlichen Rippen (17) umfasst, die von der Seitenfläche (22, 23) an der Außenkante (21) hervorstehen.

4. Vorrichtung nach einem der vorstehenden Ansprüche, wobei die Innenfläche (20) einen Durchmesser zwischen 15 und 45 mm aufweist; wobei der Durchmesser der Innenfläche (20) vorzugsweise zwischen 20 und 30 mm beträgt.

5. Vorrichtung nach einem der vorstehenden Ansprüche, wobei die zylindrische Außenfläche (22) einen Durchmesser aufweist, der mit dem größeren Durchmesser der kegelstumpfförmigen Außenfläche (23) zusammenfällt.

6. Vorrichtung nach einem der vorstehenden Ansprüche, wobei der Kernbohrungsabschnitt (19*) eine Verlängerung entlang der zweiten Achse (X') zwischen 15 und 30 mm aufweist, um Proben (C) einer einzelnen Schicht zwischen den Schichten zu isolieren, welche die Schneedecke definieren.

7. Vorrichtung nach einem der vorstehenden Ansprüche und umfassend ein Glied (29) zum Komprimieren der Schneedeckenprobe (C), das einen zylindrischen Körper (30) aufweist, der dazu bestimmt ist, innerhalb des Probenahmeelements (18) zu gleiten.

8. Vorrichtung nach Anspruch 7, wobei das Kompressionsglied (29) eine erste Kraftmessdose zum Messen der Kompressionskraft umfasst, die auf die Schneedeckenprobe (C) ausgeübt wird.

9. Vorrichtung nach einem der vorstehenden Ansprüche, wobei der Schlitten (5) eine zweite Kraftmessdose zum Messen der Scherfestigkeit der Schneedeckenprobe (C) umfasst.

10. Vorrichtung nach einem der vorstehenden Ansprüche, wobei die Struktur (2) ein Zwischenstück (12) umfasst, das mit der Platte (7) verbunden ist und mit einer zweiten Durchgangsöffnung (15) versehen ist, die koaxial zu der ersten Achse (X) ist.

11. Vorrichtung nach Anspruch 10, wenn abhängig von Anspruch 2, wobei die Nuten (16) in dem Zwischenstück (12) ausgebildet sind.

12. Vorrichtung nach einem der vorstehenden Ansprüche, wobei während des Schneedeckenproben-(C) Analyseschritts das Ende des Analyseabschnitts (19**) und der Schlitten (5) durch einen Zwischenraum mit Abmessungen zwischen 0,1 und 3 mm getrennt sind.

13. Verfahren, um eine Schneedeckenprobe (C) einer Analyse mittels einer tragbaren Vorrichtung (1) zu unterziehen, die nach einem der Ansprüche 1 bis 12 verwirklicht ist und Folgendes umfasst:
einen Schritt zur Entnahme, mittels des Kernbohrungsabschnitts (19*), einer Schneedeckenprobe (C);
einen Schritt zum Einführen des Probenahmeelements (18) in die Durchgangsöffnung (11);
einen Schritt des Aufbringens eines Drucks auf die Schneedeckenprobe (C), um sie aus einer Entnahmeposition, in der die Probe (C) vollständig im Inneren des Probenahmeelements (18) beherbergt ist, in eine Analyseposition zu bewegen, in der die Probe (C) teilweise innerhalb des Probenahmeelements (18) und teilweise innerhalb des Sitzes (25) beherbergt ist;
einen Schritt zum Steuern der Bewegung des Schlittens (5) mittels der Betätigungsvorrichtung (32); und
einen Schritt zum Messen der Scherfestigkeit der Schneedeckenprobe (C).

14. Verfahren nach Anspruch 13, wobei der Einführschritt für das Einführen des Kernbohrungsabschnitts (19*) in die Durchgangsöffnung (11) vorgesehen ist.

15. Verfahren nach Anspruch 14, wobei der Einführschritt für das Einführen des Analyseabschnitts (19**) in die Durchgangsöffnung (11) vorgesehen ist.

16. Verfahren nach einem der Ansprüche 13 bis 15, wobei keine weiteren Schritte für die Verarbeitung oder Übertragung der Schneedeckenprobe (C) zwischen dem Entnahmeschritt und dem Einführschritt vorgesehen sind.

## Revendications

1. Appareil portatif (1) pour soumettre un échantillon de manteau neigeux (C) à l'analyse, comprenant
une structure (2) pourvue à son tour de :
- une structure de guidage fixe (3) définie par deux guides linéaires (4) ;
- une plaque (7) pourvue d'une première ouverture traversante (11) ayant une symétrie cylindrique autour d'un premier axe (X) ;
- un chariot (5) qui coulisse sur les guides linéaires (4) et qui est pourvu d'un siège (25) qui est destiné à loger, au moins partiellement, l'échantillon de manteau neigeux (C) et faisant face, en utilisation, à la première ouverture traversante (11) ;
un dispositif d'actionnement (32) de chariot (5) ; et
un élément d'échantillonnage extractible (18), mis en oeuvre au moyen d'un corps tubulaire (19) ayant une surface interne (20) avec une symétrie cylindrique autour d'un second axe (X') qui définit un siège pour recevoir l'échantillon de manteau neigeux (C) ;
l'appareil est **caractérisé en ce que** l'élément d'échantillonnage extractible (18) est divisé en une partie de carottage (19*) et une partie d'analyse (19**) ; dans lequel la partie de carottage (19*) a une surface tronconique externe (23) qui est adaptée pour pénétrer dans le manteau neigeux afin d'isoler et retenir l'échantillon (C) dans le siège défini par la surface interne (20) ; et dans lequel la partie d'analyse (19**) a une surface cylindrique externe (22) ayant un diamètre qui se rapproche sensiblement du diamètre de la première ouverture traversante (11).

2. Appareil selon la revendication 1, dans lequel l'élément d'échantillonnage (18) comprend un certain nombre de nervures latérales (17) qui font saillie depuis la surface latérale (22, 23) ; et dans lequel la structure (2) comprend un certain nombre de rainures (16), dont chacune est destinée à recevoir une nervure latérale (17) respective pendant l'étape d'analyse de l'échantillon de manteau neigeux (C).

3. Appareil selon la revendication 1 ou 2, dans lequel la partie de carottage (19*) et la partie d'analyse (19**) sont jointes l'une à l'autre au niveau d'un bord externe (21) ; et dans lequel l'élément d'échantillonnage (18) comprend un certain nombre de nervures latérales (17) qui font saillie depuis la surface latérale (22, 23) au niveau du bord externe (21).

4. Appareil selon l'une quelconque des revendications précédentes, dans lequel la surface interne (20) a un diamètre compris entre 15 et 45 mm ; de préférence le diamètre de la surface interne (20) est compris entre 20 et 30 mm.

5. Appareil selon l'une quelconque des revendications précédentes, dans lequel la surface cylindrique externe (22) a un diamètre qui coïncide avec le plus grand diamètre de la surface tronconique externe (23).

6. Appareil selon l'une quelconque des revendications précédentes, dans lequel la partie de carottage (19*) présente une extension le long du second axe (X') comprise entre 15 et 30 mm, de manière à isoler les échantillons (C) d'une couche unique par rapport à entre les couches qui définissent le manteau neigeux.

7. Appareil selon l'une quelconque des revendications précédentes et comprenant un élément (29) pour la compression de l'échantillon de manteau neigeux (C) ayant un corps cylindrique (30) qui est destiné à coulisser à l'intérieur de l'élément d'échantillonnage (18).

8. Appareil selon la revendication 7, dans lequel l'élément de compression (29) comprend une première cellule de mesure pour mesurer la force de compression exercée sur l'échantillon de manteau neigeux (C).

9. Appareil selon l'une quelconque des revendications précédentes, dans lequel le chariot (5) comprend une seconde cellule de mesure pour mesurer la résistance au cisaillement de l'échantillon de manteau neigeux (C).

10. Appareil selon l'une quelconque des revendications précédentes, dans lequel la structure (2) comprend un adaptateur (12) qui est relié à la plaque (7) et pourvu d'une seconde ouverture traversante (15) qui est coaxiale au premier axe (X).

11. Appareil selon la revendication 10, lorsqu'elle dépend de la revendication 2, dans lequel les rainures (16) sont formées dans l'adaptateur (12).

12. Appareil selon l'une quelconque des revendications précédentes, dans lequel pendant l'étape d'analyse de l'échantillon de manteau neigeux (C), l'extrémité de la partie d'analyse (19**) et le chariot (5) sont séparés par un interstice de dimensions comprises entre 0,1 et 3 mm.

13. Procédé de soumission d'un échantillon de manteau neigeux (C) à l'analyse au moyen d'un appareil portatif (1) mis en oeuvre selon l'une quelconque des revendications 1 à 12 et comprenant :
une étape de retrait, au moyen de la partie de carottage (19*), d'un échantillon de manteau neigeux (C) ;
une étape d'insertion de l'élément d'échantillonnage (18) à l'intérieur de l'ouverture traversante (11);
une étape d'application d'une pression sur l'échantillon de manteau neigeux (C) afin de le déplacer depuis une position de retrait, dans laquelle l'échantillon (C) est entièrement logé à l'intérieur de l'élément d'échantillonnage (18), vers une position d'analyse, dans laquelle l'échantillon (C) est partiellement logé à l'intérieur de l'élément d'échantillonnage (18) et partiellement à l'intérieur du siège (25) ;
une étape de commande, au moyen du dispositif d'actionnement (32), du mouvement du chariot (5) ; et
une étape de mesure de la résistance au cisaillement de l'échantillon de manteau neigeux (C).

14. Procédé selon la revendication 13, dans lequel l'étape d'insertion permet l'insertion de la partie de carottage (19*) à l'intérieur de l'ouverture traversante (11).

15. Procédé selon la revendication 14, dans lequel l'étape d'insertion permet l'insertion de la partie d'analyse (19**) à l'intérieur de l'ouverture traversante (11).

16. Procédé selon l'une quelconque des revendications 13 à 15, dans lequel aucune étape supplémentaire n'est prévue pour le traitement ou le transfert de l'échantillon de manteau neigeux (C) entre l'étape de retrait et l'étape d'insertion.
